# EUROPEAN PATENT SPECIFICATION

(11) **EP 2 978 461 B1**
(45) Date of publication and mention of the grant of the patent: **24.07.2024**
(21) Application number: 14715254.0
(22) Date of filing: 31.03.2014
(51) Int. Cl.: A61L 9/12, A61L 9/04, A63J 25/00

(54) **OLFACTORY DEVICE**
OLFAKTORISCHE VORRICHTUNG
DISPOSITIF OLFACTIF

(30) Priority: 29.03.2013 US 201361806460 P
(43) Date of publication of application: 03.02.2016
(73) Proprietor: Givaudan SA, 1214 Vernier (CH)
(72) Inventor: BURLAND, Matthew Charles, Cincinnati, Ohio 45236 (US); KAISER, Joseph Andrew, Alexandria, Kentucky 41001 (US)
(74) Representative: Global Patents
(86) International application number: PCT/EP2014/056420
(87) International publication number: WO 2014/154909

(56) References cited:
- EP-A2- 1 574 017
- WO-A1-2013/057458
- WO-A1-99/01793
- US-A- 5 949 522
- US-A1- 2003 206 834
- US-A1- 2005 147 523
- US-A1- 2005 195 366
- US-B1- 6 783 084

## Description

This disclosure relates to a method of disseminating vapour-phase substances to the nasal cavities of an individual user as part of a media presentation.

It has been suggested that aroma substances, that is, substances that are either in vapour form or can easily be converted to vapour form and in which form provide a desired odour, may be used in media presentations to enhance the verisimilitude of the experience. Such a system is described in, for example, US6783084, which describes the supply to an individual user. It has also been proposed to supply such substances in cinemas and theatres. A number of such systems have been suggested and even made and operated, but all suffer from the drawbacks that, in an auditorium, even a small one, large volumes of aroma substances are required, and substantial ventilation systems are required both to blow the substances into the auditorium and to extract them in sufficiently short time for the next substances effectively to be used. In addition, achieving a uniform effect for an entire audience, even quite a small one, is a daunting technical challenge.

A different approach was taken by US6371165, in which vapour-phase substances were supplied to the nasal cavities of an individual user via a conduit. This allowed not only for the use of small quantities of substance, but also of rapid changes in substance to match the viewed programme. This versatility allowed such devices to be used not only in auditoriums, but also in domestic settings, for example, in conjunction with DVD players and computer downloads. The system may be computer-controlled and may utilize sophisticated algorithms to deliver its mixtures.

Such a device is not without its problems. For example, while much smaller than the prior devices, it is still of substantial size. In addition, there is no mention of the length of the tubing that takes the scent from the device to the nasal passages. This is an important factor; if a conduit is too long, there is often observed a fractionation effect, in which various components of the desired aroma move at different rates and reach the nasal cavity at different times, thus spoiling a carefully-calculated desired olfactory effect.

It has now been found that these problems can be substantially and even completely overcome by means of a device that allows a hitherto unachievable miniaturisation, thus allowing the manufacture of devices that are easily incorporated into seats, or even made wearable. The disclosure therefore provides a non-therapeutic aroma substance delivery device adapted to deliver an aroma substance exclusively to the nasal cavities of an individual, comprising the following elements:
(i) a source of carrier gas flow;
(ii) regulating means which receives the carrier gas flow and regulates its passage through a plurality of channels;
(iii) downstream of the regulating means a plurality of aroma substance-containing cartridges, one being associated with each channel, and
(iv) disseminating means located in close proximity to the cartridges and adapted to deliver the individual aroma substances from the cartridges to nasal cavities by means of a conduit;
there being positioned in each channel on the upstream side and in close proximity to the origin of the channel a flow restrictor, the flow restrictor being configured such that the pressure drop across the flow restrictor is higher than the pressure drop across the rest of the device.

The source of carrier gas may be any suitable source of gas. For example, the carrier gas may be air, and the source may be a compressor. Alternatively, the source may be a pressurised cylinder of gas.

The gas flow is conveyed to a regulating means. This means is typically a device that comprises a plurality of channels adapted to convey the gas to cartridges containing aroma substance, one cartridge per channel. Thus, by opening and closing channels and maintaining these openings and closings for predetermined times, the regulating means can determine which aroma substances and how much thereof are conveyed and when, and thus alter the nature of the aroma perceived by a user. Devices that can fulfil this function are well known in the field of orthonasal testing, for example the MiniVAS^{™} device of Givaudan. The operation of the regulating means may be remote, for example, by computer either wired to the regulating device or operating it wirelessly. Thus, the operation of the individual channels may be programmed, such that particular types and quantities of aroma substances may be delivered in synchronisation with an audio-visual event, such as a film or slide show. This aspect is further described hereinunder.

In a particular embodiment, such a regulating means is capable of regulating the carrier gas flow, suitably charged with aroma substances, to a plurality of disseminating means. Thus, such a means may, for example, provide identical aroma substances to a plurality of users simultaneously, for example, patrons in a cinema.

The cartridges of aroma substance from which the desired aroma is created are capable of containing the desired aroma substances and releasing them when impinged upon by a carrier gas. The cartridge comprises a reservoir containing an aroma substance and being provided with inlet and outlet channels to permit ingress of carrier gas into the reservoir and egress of aroma substance-containing carrier gas from the reservoir, the channels being defined by capillaries having internal diameter and length dimensions sufficient to act as closure means to prevent, or substantially prevent, leakage of aroma substance from the reservoir into a head space external of the cartridge when carrier gas flow is interrupted. Such a cartridge is described in European Patent 1523340. It has the advantages that it can be small and very slim (about credit card-sized) and that it need no valve arrangements, merely a supply of carrier gas to entrain the substance from the cartridge and means of directing and regulating this. Such cartridges are easily replaced when empty.

The aroma substances that are entrained by the gas flow from the cartridges are conveyed by the carrier to a disseminating means via a conduit. The nature of the disseminating means may be any disseminating means capable of delivering a vapour-phase substance to the nasal cavities of an individual. For example, it may be a small nose piece able to be worn, or it may be an open tube placed near the nasal cavities. Typical examples of suitable disseminating means are those described in the abovementioned US 6371165 and EP 1523340. Alternatively, it may be a tube mounted on a seat and capable of adjustment to be near the nose of a seat occupant. Many possible variants will occur to the skilled person. The various aroma substances may be blended prior to entering a single conduit, or each aroma substance may arrive at the disseminating means via individual conduits.

The aroma substance-containing cartridges are placed in close proximity to the disseminating means. By "close proximity" is meant that the distance between cartridge and disseminating means should be kept to a minimum. What this minimum is will be determined by the specific nature of the device and its location, but the skilled person will readily be able to determine a suitable solution in any particular case. For example, if the disseminating means is mounted on a flexible "gooseneck"-type fitting of the type frequently used for microphones), the cartridges should be as close as practicable to the mounting end of the gooseneck, for example, in a compartment of an audience seat headrest.

A further feature of the device is the presence of flow restrictors upstream of the cartridges with respect to the gas flow and in close proximity to the origin of the channel. By "origin of the channel" is meant that point in the apparatus where the channel becomes an individual channel. In a typical arrangement, this will involve a manifold that receives the gas flow, and from which emerge the desired plurality of channels, one per cartridge. In such a case, the flow restrictors should be placed as close to the manifold as possible, and may form part of the manifold.

Suitable flow restrictors are well known to the art, and are commercially available from, for example, Universal Microsystems and Mott Corp. The type selected is such that the pressure drop across the flow restrictor is greater than that across the rest of the device as a whole. In such a configuration, they are rate-determining, and they do away with the need for complicated and expensive mass-flow controllers.

As previously mentioned, the device may additionally comprise control means that control its entire operation, including the selection and quantity of aroma substance at any desired time by means of operation of the regulating device. Control means capable of such operations are well known to the art, and are available as off-the-shelf hardware, or can be made or modified by a skilled person.

The software required for such operation and its generation of the necessary operational signals are also well known to the art and a skilled programmer can easily provide suitable programming for any use.

The control means may be located in any convenient location, for example, within individual cinema seats, or in close proximity to a group of seats, or it may be in a single central location, giving instructions to a plurality of regulating devices by wire or wireless means. It may be manually operated, or it may be a completely automated unit into which all necessary inputs are programmed and which generates all the necessary inputs at the appropriate times and communicates them to the regulating means.

The device is particularly useful in conjunction with audiovisual presentations for entertainment and education, for example, movies, slide shows, theatrical presentations, and the like. It is possible to use the device in live presentations, such as plays, operas and ballets. In such a case, it is generally preferred to operate the device manually from a central location. However, in a particular embodiment, in which visual projection of images is involved, a suitably-programmed central computer can coordinate all aspects of a presentation, coordinating visual, audio and olfactory elements, to provide a complete and perfectly-timed sensory experience.

The device may be operated centrally in a theatre, such that all patrons may receive the same vapour-phase substance at the same, appropriate time. As hereinabove described, this may be done manually, or it may be completely automatic, programmed to react to signals that may be transmitted by cable or wirelessly. In a domestic situation, for example, with a DVD player, the appropriate signals may be encoded on the DVD, and these then communicated to the device by any suitable means, such that the appropriate vapour-phase substance is released at the appropriate time and in the appropriate quantity. In the case of a television programme or a digital download from a computer, the appropriate signals may be encoded according to a predetermined standard format.

The device hereinabove described is very useful in conjunction with entertainment venues such as cinemas, theatres, pubic lectures and the like. It may also be used in domestic situations, in conjunction with television, DVD players and digital download material. The disclosure therefore provides means for the provision of individual olfactory sensation as part of an audiovisual educational or entertainment presentation, comprising a device as hereinabove described.

There is also provided a method of providing to an individual an olfactory sensation corresponding to a simultaneously-perceived visual and/or auditory sensation, comprising supplying to the nasal passages of the individual an aroma substance, the aroma substance being supplied by operation of a device as hereinabove described.

There is further provided a complete sensory audio/video/olfactory educational or entertainment experience in which the senses of sight, hearing and smell are simultaneously stimulated in a coordinated manner, comprising the provision of audio/visual apparatus having coordinated visual and aural elements, plus additional olfactory elements coordinated with the visual and/or aural elements, the coordinated olfactory elements being provided by an aroma substance delivery device as hereinabove described.

The disclosure is further described with reference to the accompanying drawings, which depict particular embodiments of the device, and which are not meant to be in any way limiting.

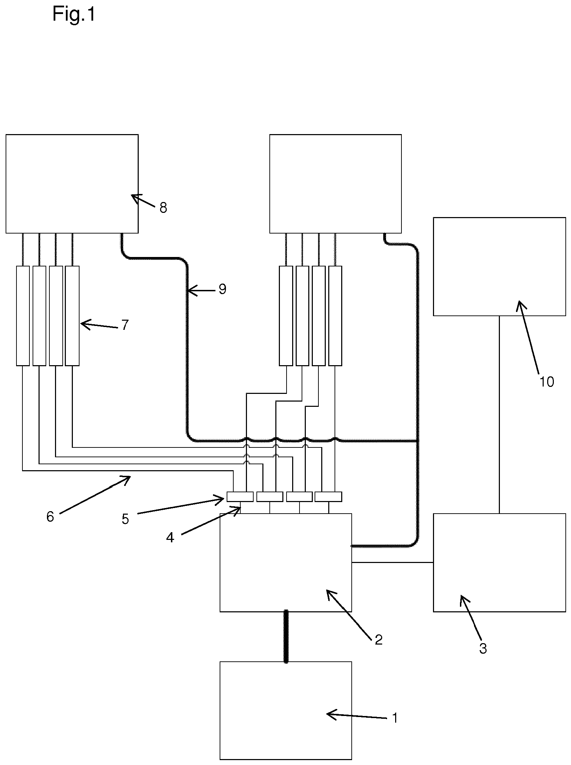

Figure 1 is a schematic representation of a device as hereinabove described, for use with an audiovisual system with multiple audience places.

For simplicity of illustration, the device of Fig. 1 is depicted as having only two disseminating means and 4 aroma substance cartridges each. In reality, the device would have as many disseminating means as there are audience places and as many cartridges as are needed for the desired range of olfactory sensations in the audiovisual display.

An air compressor 1 is attached to a regulating means 2. The regulating means is a MiniVAS^{™} device (ex Givaudan). This regulating means contains regulating valves controlling the passage of air from the compressor to a plurality of primary channels 4, one per aroma substance. The regulating means is controlled by a computer 3, which is programmed with the information as to which aroma substances are to be released in which proportions at which time and for which time period to provide the desired olfactory sensation.

The primary channels 4 extend from the regulating means 2 to a series of manifolds 5. From these manifolds extend a series of secondary channels 6, one secondary channel per audience place. Within each secondary channel 6 at the point where it exits from the manifold 5 is placed a flow restrictor, configured such that the pressure drop across the flow restrictor is higher than the pressure drop across the rest of the device.

Each secondary channel 6 leads to an aroma substance cartridge 7, these being of the type described in European Patent 1523340, and each containing an individual aroma substance. From the cartridges 7, the secondary channels extend further to a disseminating means 8, in this case an individual nose-piece, placed such that the distance between the cartridges and the nose-piece is small. Each nose-piece is additionally equipped with a further air channel 9, extending independently from the regulating means 2, and also under the control of the computer 3. This is adapted to communicate directly to the nose-piece 8 additional air, for the purposes of suitable dilution or for clearing an odour from the nose-piece in preparation for the next odour.

In practice, all instructions are programmed into the computer 3, which also controls an audiovisual projector 10. As the display continues, the computer activates the regulating means 2 to deliver, in conjunction with the cartridges 7, at the appropriate time and in the appropriate quantity and duration, olfactory sensations to accord with what the individual wearers are observing, courtesy of the projector.

## Claims

1. A non-therapeutic aroma substance delivery device adapted to deliver an aroma substance exclusively to the nasal cavities of an individual, comprising the following elements:
(i) a source of carrier gas flow (1);
(ii) regulating means (2) which receives the carrier gas flow, the regulating means comprising a plurality of channels (4, 6) through which the gas flows and through which the gas flow is regulated by the regulating means;
(iii) downstream of the regulating means one aroma substance-containing cartridge (7), one on each channel, and
(iv) disseminating means (8) located in close proximity to the cartridges and adapted to deliver the individual aroma substances from the cartridges to nasal cavities by means of a conduit;
**characterised in that** there is positioned in each channel on the upstream side and in close proximity to the origin of the channel a flow restrictor, the flow restrictor being configured such that the pressure drop across the flow restrictor is higher than the pressure drop across the rest of the device;
and
**in that** each aroma substance-containing cartridge (7) comprises a reservoir containing an aroma substance and inlet and outlet channels to permit ingress of carrier gas into the reservoir and egress of aroma substance-containing carrier gas from the reservoir, the channels being defined by capillaries having internal diameter and length dimensions such that, when the carrier gas flow is interrupted, leakage of aroma substance from the reservoir into a head space external of the cartridge is below the detection level of a user.

2. Apparatus for the provision of non-therapeutic individual olfactory sensation as part of an audiovisual educational or entertainment presentation, **characterised in that** it comprises a device according to claim 1.

3. A non-therapeutic method of providing to an individual a non-therapeutic olfactory sensation corresponding to a simultaneously-perceived visual and/or auditory sensation, comprising supplying to the nasal passages of the individual an aroma substance, **characterised in that** the aroma substance is supplied by operation of a device according to claim 1.

4. Use of an aroma substance delivery device according to claim 1 in the provision of coordinated olfactory elements in the delivery of a complete sensory audio/video/olfactory educational or entertainment experience in which the senses of sight, hearing and smell are simultaneously stimulated in a coordinated manner, comprising the provision of audio/visual apparatus having coordinated visual and aural elements, plus additional olfactory elements coordinated with the visual and/or aural elements.

## Patentansprüche

1. Nicht-therapeutische Duftstoffabgabeeinrichtung, die dazu angepasst ist, einen Duftstoff ausschließlich in die Nasenhöhlen einer Person abzugeben, umfassend die folgenden Elemente:
(i) eine Quelle von Trägergasstrom (1);
(ii) ein Regelungsmittel (2), das den Trägergasstrom empfängt, wobei das Regelungsmittel eine Vielzahl von Kanälen (4, 6) umfasst, durch die das Gas strömt und durch die der Gasstrom durch das Regelungsmittel geregelt wird;
(iii) stromabwärts des Regelungsmittels eine duftstoffhaltige Patrone (7), eine auf jedem Kanal, und
(iv) Verteilungsmittel (8), die sich in unmittelbarer Nähe der Patronen befinden und dazu angepasst sind, die einzelnen Duftstoffe aus den Patronen mittels über eine Leitung in die Nasenhöhlen abzugeben;
**dadurch gekennzeichnet, dass** in jedem Kanal auf der stromaufwärtigen Seite und in unmittelbarer Nähe des Ursprungs des Kanals ein Durchflussbegrenzer angeordnet ist, wobei der Durchflussbegrenzer so ausgelegt ist, dass der Druckabfall über den Durchflussbegrenzer höher als der Druckabfall über den Rest der Vorrichtung ist; und
dass jede einen Duftstoff enthaltende Patrone (7) ein einen Duftstoff enthaltendes Vorratsgefäß sowie Einlass- und Auslasskanäle umfasst, um den Eintritt von Trägergas in das Vorratsgefäß und den Austritt von einen Duftstoff enthaltendem Trägergas aus dem Vorratsgefäß zu ermöglichen, wobei die Kanäle durch Kapillaren definiert sind, deren Innendurchmesser und Länge so bemessen sind, dass bei Unterbrechung des Trägergasstroms das Austreten des Duftstoffs aus dem Vorratsgefäß in einen Leerraum außerhalb der Patrone unterhalb der Nachweisgrenze eines Benutzers liegt.

2. Vorrichtung zur Bereitstellung eines nichttherapeutischen individuellen Geruchsempfindens als Teil einer audiovisuellen Bildungs- oder Unterhaltungsdarbietung, **dadurch gekennzeichnet, dass** sie eine Einrichtung nach Anspruch 1 umfasst.

3. Nicht-therapeutisches Verfahren, um einer Person eine nicht-therapeutische Geruchsempfindung zu vermitteln, die einer gleichzeitig wahrgenommenen visuellen und/oder auditiven Empfindung entspricht, umfassend das Zuführen eines Duftstoffs zu den Nasenkanälen der Person, **dadurch gekennzeichnet, dass** der Duftstoff durch Betätigung einer Einrichtung nach Anspruch 1 zugeführt wird.

4. Verwendung einer Duftstoffabgabeeinrichtung nach Anspruch 1 bei der Bereitstellung von koordinierten olfaktorischen Elementen bei der Abgabe eines vollständigen sensorischen Audio-/Video-/olfaktorischen Bildungs- oder Unterhaltungserlebnisses, bei dem der Seh-, Hör- und Geruchssinn gleichzeitig koordiniert stimuliert wird, umfassend die Bereitstellung einer auditiven/visuellen Vorrichtung mit koordinierten visuellen und auditiven Elementen sowie zusätzlichen olfaktorischen Elementen, die mit den visuellen und/oder auditiven Elementen koordiniert sind.

## Revendications

1. Dispositif de distribution de substance aromatique non thérapeutique conçu pour distribuer une substance aromatique exclusivement aux cavités nasales d'un individu, comprenant les éléments suivants :
(i) une source de flux de gaz porteur (1) ;
(ii) des moyens de régulation (2) qui reçoivent le flux de gaz porteur, les moyens de régulation comprenant une pluralité de canaux (4, 6) à travers lesquels le gaz s'écoule et à travers lesquels le flux de gaz est régulé par les moyens de régulation ;
(iii) en aval des moyens de régulation, une cartouche contenant une substance aromatique (7), une sur chaque canal, et
(iv) des moyens de dissémination (8) situés très près des cartouches et conçus pour distribuer les substances aromatiques individuelles depuis les cartouches dans les cavités nasales au moyen d'un conduit ;
**caractérisé en ce que** dans chaque canal sur le côté amont et très près de l'origine du canal est positionné un restricteur de flux, le restricteur de flux étant conçu de telle sorte que la chute de pression dans le restricteur de flux est supérieure à la chute de pression à travers le reste du dispositif ; et
**en ce que** chaque cartouche contenant une substance aromatique (7) comprend un réservoir contenant une substance aromatique et des canaux d'entrée et de sortie pour permettre l'admission de gaz porteur dans le réservoir et la sortie de gaz porteur contenant une substance aromatique depuis le réservoir, les canaux étant définis par des capillaires ayant un diamètre interne et des dimensions de longueur tels que, lorsque le flux de gaz porteur est interrompu, une fuite de substance aromatique du réservoir dans un espace de tête externe à la cartouche est inférieure au niveau de détection d'un utilisateur.

2. Appareil de fourniture d'une sensation olfactive individuelle non thérapeutique dans le cadre d'une présentation audiovisuelle éducative ou de divertissement, **caractérisé en ce qu'**il comprend un dispositif selon la revendication 1.

3. Procédé non thérapeutique de fourniture à un individu d'une sensation olfactive non thérapeutique correspondant à une sensation visuelle et/ou auditive perçue simultanément, comprenant la fourniture aux cavités nasales de l'individu d'une substance aromatique, **caractérisé en ce que** la substance aromatique est fournie par le fonctionnement d'un dispositif selon la revendication 1.

4. Utilisation d'un dispositif de distribution de substance aromatique selon la revendication 1 dans la fourniture d'éléments olfactifs coordonnés dans la distribution d'une expérience sensorielle complète audio/vidéo/olfactive éducative ou de divertissement dans laquelle les sens de la vue, de l'ouïe et de l'odorat sont stimulés simultanément de manière coordonnée, comprenant la fourniture de l'appareil audio/visuel ayant des éléments visuels et auditifs coordonnés, plus les éléments olfactifs coordonnés avec les éléments visuels et/ou auditifs.
